Europäisches Patentamt

(19) European Patent Office    (11) Numéro de publication: **0 129 630**

Office européen des brevets    **B1**

(12)    # FASCICULE DE BREVET EUROPÉEN

(45) Date de publication du fascicule du brevet:    (51) Int. Cl.⁴: **G 01 N 33/02,** B 07 C 5/34,
**20.05.87**    **G 01 N 21/41**

(21) Numéro de dépôt: **83430019.6**

(22) Date de dépôt: **24.06.83**

(54) **Dispositif pour sélectionner en continu des fruits, en fonction de leur teneur en sucre.**

(43) Date de publication de la demande:
**02.01.85 Bulletin 85/1**

(45) Mention de la délivrance du brevet:
**20.05.87 Bulletin 87/21**

(84) Etats contractants désignés:
**AT BE CH DE GB IT LI LU NL SE**

(56) Documents cité:
**FR-A-982 346**
**FR-A-2 053 730**
**FR-A-2 209 305**
**US-A-3 218 552**

(73) Titulaire: **Giraud, Charles, Quartier Saint Ferréol,
F-84460 Cheval Blanc (FR)**

(72) Inventeur: **Giraud, Charles, Quartier Saint Ferréol,
F-84460 Cheval Blanc (FR)**

(74) Mandataire: **Moretti, René, C/O Cabinet BEAU DE
LOMENIE 14, rue Raphael, F-13008 Marseille (FR)**

LIBER, STOCKHOLM 1987

## Description

La présente invention a pour objet un dispositif pour sélectionner en continu des fruits en fonction de leur teneur en sucre.

Le secteur technique de l'invention est celui des appareillages pour contrôler la teneur en sucre des fruits frais et se rapporte plus particulièrement à de tels appareillages utilisés notamment dans les stations de calibrage et de conditionnement des fruits.

Om sait que les qualités gustatives des fruits frais notamment sont intimememt liées à leur teneur en sucre.

L'objectif à atteindre est la sélection en continu avant leur calibrage et leur conditionnement, des fruits frais d'une même variété, tels que des melons, pastèques, ananas et autres produits similaires, en fonction de leur teneur en sucre, comparée à au moins un seuil au-dessus duquel les fruits présentent des qualités gustatives requises et sont dirigés vers un stockage ou une station de calibrage et de conditionnement, et pour écarter et rejeter les fruits dont la teneur en sucre est inférieure audit seuil et ne présentent pas lesdites qualités.

Cet objectif est atteint par le dispositif pour sélectionner en continu des fruits d'une même variété en fonction de leur teneur en sucre, comportant, en combinaison:

- des moyens pour déplacer les fruits et pour les présenter un à un devant un poste pour prélever dans chaque fruit une carotte de pulpe,
- des moyens pour extraire de la pulpe le jus qu'elle contient;
- des moyens pour analyser le jus et pour déterminer sa teneur en sucre et des moyens pour comparer le résultat de chaque analyse à au moins un seuil prédétermimé correspodant à la teneur en sucre minimale au-dessous duquel les fruits sont rejetés;
- des moyens pour évacuer les fruits dont la teneur en sucre se situe au-dessous dudit seuil et des moyens pour diriger les fruits sélectionnés vers un stockage ou une station de calibrage et de conditionnement.

Le dispositif comporte, en outre, des moyens pour replacer la pulpe dans le fruit après l'extraction du jus.

Dans un mode de réalisation les fruits sont déplacés par un convoyeur et le dispositif comporte un détecteur de présence situé sur le trajet des fruits et à proximité de moyens pour immobiliser temporairement les fruits, consistant en une plaque d'appui fixée le long d'un des bords latéraux du convoyeur et d'un organe de blocage porté par la tige mobile d'un vérin pour être déplacée devant et perpendiculairement à ladite plaque d'appui et transversalement au convoyeur pour bloquer un à un les fruits contre la plaque d'appui.

Ladite plaque est percée pour permettre le passage desdits moyens pour prélever une carotte de pulpe, lesquels moyens se composent d'une gouge constituée par un tube dont le bord périphérique de son extrémité libre est affûté en biseau pour pouvoir pénétrer dans le fruit et prélever une partie de pulpe ainsi que la peau du fruit, laquelle gouge est portée par la tige mobile d'un vérin à double effet et se déplace parallèlement au-dessus du tapis du convoyeur.

Le dispositif comporte, en outre, une tige-poussoir montée coulissante dans ladite gouge, laquelle tige-poussoir est portée par la tige mobile d'un vérin à double effet pour se déplacer dans la gouge coaxialement à celle-ci pour, d'une part, faire sortir de la gouge une partie de la carotte pour extraire le jus qu'elle contient et, d'autre part, replacer, après l'extraction du jus, la carotte dans le trou pratiqué dans le fruit lors du prélèvement de la carotte. En position "rentrée" de la tige mobile desdits vérins, la position relative de la tige et de la gouge est telle que la pulpe d'une partie de la carotte est maintenue momentanément au-dessus du prisme d'un réfractomètre.

Le dispositif comporte au-dessus dudit prisme, un pressoir constitué par un vérin à double effet dont la tige mobile comporte à son extrémité libre un piston destiné à presser la pulpe du fruit pour extraire le jus, lequel piston se déplace dans le sens vertical pour s'arrêter en partie basse à proximité dudit prisme.

Ledit piston est percé dans le sens transversal pour permettre le passage de la gouge pour replacer la carotte dans le fruit après l'extraction du jus. Le dispositif comporte encore des moyens pour rincer le prisme du réfractomètre consistant en une vanne télécommandée comportant un gicleur dirigé vers ledit prisme et dont l'extrémité se situe à proximité de celui-ci, laquelle vanne est reliée à un circuit d'eau sous pression pour délivrer et projeter sur le prisme un jet d'eau après chaque analyse.

Il comporte également des moyens pour nettoyer et sécher le prisme du réfractomètre, consistant en une vanne télécommandée comportant un gicleur dirigé vers ledit prisme et dont l'extrémité se situe à proximité de celui-ci, laquelle vanne est reliée à un circuit d'air comprimé pour délivrer et souffler sur le prisme un jet d'air après le rinçage.

Les moyens pour écarter les fruits dont la teneur en sucre n'atteint pas ledit seuil, consistent en au moins un vérin dont la tige mobile comporte à son extrémité libre un poussoir, lequel poussoir se déplace transversalement et parallèlement au-dessus du convoyeur pour pousser les fruits sur un plan incliné débouchant dans un bac de réception.

Le dispositif selon l'invention dont les vérins comportent des capteurs de position et sont pilotés se caractérise également en ce que le détecteur de présence, les capteurs de position et les pilotes, les vérins, le réfractomètre, les vannes d'eau et d'air pour rincer et nettoyer et sécher le prisme du réfractomètre et le vérin d'éjection sont reliés à une unité de commande qui reçoit des signaux transmis par les différents appareils composant l'installation, qui compare l'analyse du réfractomètre par rapport à au moins un seuil et en fonction d'un programme préétabli, délivre des signaux de sortie pour commander lesdits appareils selon un cycle de fonctionnement.

Le résultat de l'invention est une installation pour sélectionner les fruits frais d'une même variété en fonction de leur teneur en sucre, pour diriger les fruits qui présentent des qualités gustatives requises vers un stockage ou une station de calibrage et de conditionnement en vue de leur diffusion dans le commerce et de leur consommation.

Le dispositif selon l'invention est entièrement automatique et permet de tester et sélectionner en continu diverses variétés de fruits. Il est programmable et selon l'invention, le réfractomètre électronique permet de faire, en continu et au fur et à mesure que les fruits défilent devant le poste de prélèvement d'une partie de pulpe, l'analyse du taux de sucre du jus extrait par pression. Les moyens mis en oeuvre permettent ensuite de comparer cette analyse à un ou plusieurs seuils prédéterminés pour faire la sélection des fruits ayant les qualités gustatives requises et pour rejeter ceux qui n'ont pas ces qualités.

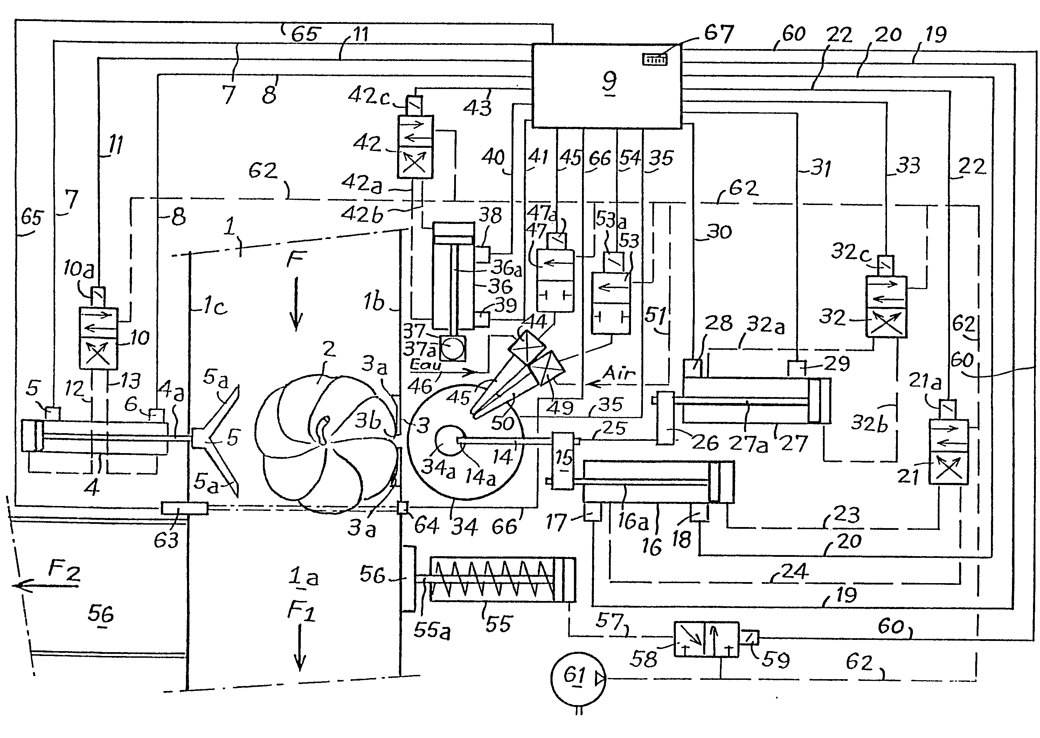

D'autres avantages et les caractéristiques de l'invention ressortiront encore à la lecture de la description suivante d'un mode de réalisation d'un dispositif selon l'invention dont la figure unique est un schéma d'une installation à fonctionnement électropneumatique pilotée par une centrale de commande électronique.

Selon un mode de réalisation, une installation pour sélectionner les fruits frais en fonction de leur teneur en sucre se compose tel qu'illustré sur le dessin annexé, d'un convoyeur 1, par exemple un transporteur à tapis dont le brin porteur 1a s'étend dans un plan horizontal. Les produits à tester, par exemple des fruits frais tels que des melons 2, sont disposés sur ledit tapis 1a, lequel les déplace dans le sens de la flèche F. Le poste pour tester la teneur en sucre des fruits est situé latéralement au convoyeur 1 et comporte un dispositif de blocage, pour immobiliser les fruits momentanément pour permettre la phase de prélèvement de la pulpe des fruits et l'analyse du jus qu'elle contient. Les moyens pour immobiliser les fruits 2 se composent d'une plaque d'appui 3 fixée au châssis du convoyeur 1, laquelle plaque est disposé, à proximité d'un des bords latéraux du convoyeur, par exemple celui 1b situé à droite sur le dessin et s'étend perpendiculairement au brin porteur du tapis 1a. La plaque 3 comporte, sur ses bords latéraux, deux rebords évasés 3a dirigés du côté des fruits pour favoriser leur immobilisation. Cette plaque peut recevoir sur la face sur laquelle les fruits viennent en appui, une couche de mousse ou de tout autre matériau souple pour éviter de meurtrir les fruits 2. Devant la plaque 3 et du côté du bord opposé 1c audit bord 1b du convoyeur est monté un vérin pneumatique à double effet 4 dont la tige mobile 4a porte à son extrémité libre un organe de blocage 5 ouvert en forme de V dont les parois laterales 5a divergent à partir de la tige 4a.

L'organe 5 est d'une forme et de dimensions convenables pour lui permettre de bloquer les fruits de différentes grosseurs.

Comme la plaque d'appui 3, les faces des parois 5a venant au contact des fruits peuvent être recouvertes d'une couche de mousse ou de tout autre matériau souple pour éviter de les meurtrir.

Le vérin 4 comporte des capteurs de position électriques 5, 6 qui donnent une information sur la position de la tige mobile 4a "rentrée" ou "sortie". Ces capteurs sont reliés par des lignes 7, 8 à une unité de commande, par exemple un séquenceur électronique 9. Celui-ci est d'un type connu et peut être également du type pneumatique ou électrique. De même les capteurs 5, 6 peuvent être du type pneumatique en fonction de la technique de pilotage adoptée.

Le vérin 4 est relié à un distributeur d'air 10 commandé par un pilote électromagnétique 10a relié par une ligne au séquenceur 9. Le distributeur 10 est relié aux extrémités de la chambre du vérin 4 par deux tubes d'air 12, 13. Le vérin 4 s'étend horizontalement et transversalement au convoyeur 1, de telle sorte que sa tige mobile se déplace parallèlement au-dessus du tapis 1a. Dans le prolongement de l'axe de la tige 4a du vérin de blocage 4, s'étend un outil 14 pour prélever une carotte de pulpe dans le fruit 2 immobilisé entre les organes 5 et 3. Cet outil 14 appelé "gouge" se présente sous la forme d'un tube affûté en biseau à son extrémité libre 14a située du côté du fruit. La gouge 14 est fixée à une monture 15 portée par la tige mobile 16a d'un vérin pneumatique à double effet 16, à laquelle elle est fixée à son extrémité libre. Cette monture 15 a pour objet de déporter la gouge 14 par rapport au vérin pour des raisons qui seront exposées plus loin. La gouge 15 et la tige 16a peuvent être dans un même plan horizontal parallèle au brin porteur 1a du tapis du convoyeur 1. L'axe sur lequel sont situées la tige 4a du verin de blocage et la gouge 14 se situe à une hauteur convenable pour que la gouge puisse pénétrer à coeur dans les fruits et quelle que soit leur grosseur. Il est précisé que la position en hauteur de la gouge par rapport au brin 1a du tapis peut être réglable dans le cas où la grosseur des fruits compte tenu de leur variété, présenterait de trop grandes différences.

Pour permettre à la gouge 14 de pénétrer dans les fruits 2, la plaque d'appui 3 est percée par exemple en son centre, d'un orifice 3b d'un diamètre légèrement supérieur à celui de la gouge 14.

Le vérin 16 comporte des capteurs de position 17, 18 du genre de ceux précédemment décrits, lesquels sont reliés par des lignes 19, 20 au séquenceur 9. Le vérin 16 comporte encore un distributeur d'air 21 à pilote électromagnétique 21a, lequel est relié par une ligne 22 audit séquenceur. Le distributeur 21 est relié à la chambre des vérins 16 par deux tubes d'air 23, 24.

Dans la gouge 14 est monté coaxialement à coulissement une tige cylindrique 25 fixée à une monture 26 elle-même fixée à l'extrémité libre de la tige 27a d'un vérin pneumatique à double effet 27 pouvant être par exemple située dans le même plan que le vérin 16. La longueur de la tige 25 est telle qu'en position "sortie" des vérins 16 et 27, son extrémité se trouve sensiblement à la périphérie du fruit 2 et en position "rentrée" illustrée sur le dessin, son extrémité se trouve en retrait de l'extrémité 14a de la gouge, sur une longueur d'environ 15 mm.

La tige 25 est ainsi déportée par rapport à la tige mobile 27a du vérin 27, lequel est dans une position horizontale et est parallèle au vérin 16. On comprend qu'en fonction de la position "rentrée" ou "sortie" des vérins 16 et 27, la position relative de l'extrémité de la gouge 14 et de l'extrémité de la tige 25 varie pour assurer les fonctions du dispositif de prélèvement de la carotte et qui seront exposées plus loin.

Le vérin 27 comporte des capteurs de position 28, 29 du genre de ceux déjà cités, lesquels sont reliés au séquenceur 9 par des lignes 30, 31 et un distributeur d'air 32 relié par des conduits 32a, 32b et comportant un pilote électromagnétique 32c relié par une ligne 33 audit séquenceur 9.

En position "rentrée" du vérin 16, l'extrémité 14a de la gouge 14 s'immobilise au-dessus du prisme 34a d'un réfractomètre électronique 34, lequel est relié au séquenceur 9 par une ligne 35.

Au-dessus du prisme 34a est disposé un pressoir constitué par un vérin pneumatique à double effet 36, disposé verticalement. Pour des besoins de représentation schématique, ce vérin est représenté "à plat" sur le dessin.

Le vérin 36 porte à l'extrémité de sa tige mobile 36a un piston, par exemple cylindrique 37. La position du vérin 36 est telle que lorsque sa tige est en position "sortie", le piston se trouve à proximité du prisme 34a du réfractomètre électronique 34 c'est-à-dire à une distance de l'ordre de quelques dixièmes de millimètres.

Pour permettre le déroulement des fonctions qui sera exposé plus loin et dans un mode de réalisation non limitatif, ce piston comporte un orifice 37a le traversant diamétralement et de part en part pour permettre le passage de la gouge 14 et de la tige 25.

Comme les autres vérins, le vérin 36 comporte des capteurs de position 38, 39 reliés au séquenceur 9 par des lignes 40, 41 et un distributeur d'air 42 relié par des circuits d'air 42a, 42b, lequel distributeur est associé à un pilote électromagnétique 42c lui-même relié au séquenceur 9 par une ligne 43.

Le rinçage du prisme 34a du réfractomètre électronique 34 est obtenu au moyen d'une vanne 44 à fonctionnement pneumatique, dont le gicleur 45 est dirigé sur le prisme 34a. Cette vanne est, d'une part, reliée à un circuit d'eau sous faible pression 46 et, d'autre part, à un distributeur d'air 47 commandé par un pilote 47a, lui-même relié à un séquenceur 9 par une ligne 48. Le dispositif comporte encore des moyens pour nettoyer et pour sécher le prisme 34a du réfractomètre, qui consistent en une vanne 49 à fonctionnement pneumatique dont le gicleur 50 est dirigé sur ledit prisme 34a. Cette vanne 49 est reliée à un circuit d'air comprimé 51 et à un distributeur d'air 53 dont le pilote 53a est relié au séquenceur 9 par une ligne 54.

Les fruits 2 sélectionnés portés par le convoyeur 1 sont dirigés dans le sens de la flèche F1 vers une station de calibrage et de conditionnement (non représentée). Les fruits rejetés sont poussés dans le sens de la flèche F2 par un vérin à simple effet 55 et sont dirigés vers un bac de réception (non représenté) en empruntant un plan incliné 56.

Le vérin 55 est dans une position horizontale et sa tige mobile 55a se déplace transversalement et parallèlement au brin porteur 1a du tapis. Elle porte à son extrémité libre un organe poussoir 54 consistant par exemple en un piston cylindrique de forme aplatie. La position du vérin 55 par rapport au convoyeur 1 est telle que le piston 56 vient au contact des fruits rejetés lors de leur passage entre lui et le plan incliné 56.

Le vérin 55 est relié par un circuit d'air 57 à un distributeur d'air 58 associé à un pilote électromagnétique 59 relié par une ligne 60 au séquenceur 9.

L'air comprimé est fourni par un compresseur 61 dont le collecteur 62 est relié aux distributeurs 10, 21, 32, 42, 47, 53, 58 ainsi qu'à la vanne d'air 49. La pression de fonctionnement est de l'ordre de 5 à 6 bars.

La quantité d'eau et la pression nécessaire sont infimes, ladite quantité étant environ 1/4 de litre par heure de fonctionnement. L'eau est stockée à l'intérieur de la machine et aboutit à la vanne 44 par une tubulure 46. L'électrovanne 44 distribue la valeur de trois à quatre gouttes environ à chaque cycle. L'électrovanne 44 est alimentée par un syphon, ce qui donne une faible pression, de l'ordre de 0,5 bar, constante quel que soit le niveau du réservoir de stockage de l'eau.

Le cycle de fonctionnement de l'invention est déclenché par un capteur pouvant être une cellule photo-électrique, un capteur de proximité électrique, électronique, pneumatique ou tout autre moyen similaire.

A titre d'exemple, on a représenté sur le schéma une cellule photo-électrique composée d'un émetteur 63 et d'un récepteur 64, lesquels sont respectivement reliés au séquenceur 9 par des lignes 65,66.

La position de l'émetteur 63 et du récepteur 64 est telle que le rayon émis par l'émetteur est dirigé transversalement au convoyeur 1 et parallèlement au-dessus du brin porteur 1a du tapis sur le trajet des fruits 2. Comme cela est illustré sur le dessin, l'émetteur 63 et le récepteur 64 sont alignés et situés en avant de la plaque d'appui 3 par rapport au sens de défilement du tapis du convoyeur 1.

Le séquenceur, par exemple un automate programmable électronique, est programé pour assurer le cycle des fonctions des différents organes qui composent le dispositif.

On fixe ensuite un ou plusieurs seuils, par exemple un seuil au-dessus duquel le taux de sucre est celui d'un produit présentant des qualités gustatives requises par exemple 9 Brix et on programme cette valeur dans le réfractomètre 34, lequel transmet l'information au séquenceur 9.

Le cycle de fonctionnement du dispositif selon l'invention est le suivant:

Au départ, on actionne une commande 67 pour effectuer la mise à zéro de l'installation.

Les fruits sont déplacés par le convoyeur 1 et sont acheminés vers le poste de blocage et d'analyse de la teneur en sucre.

## Phase 1.

Dès que le fruit 2 entre dans le champ de la cellule photoélectrique 63, 64, le cycle de fonctionnement de l'installation est déclenché.

## Phase 2.

Le vérin 4 est actionné et sa tige mobile 4a se déplace du côté de la plaque d'appui 3 en insérant le fruit entre celle-ci et l'organe de blocage 5, immobilisant le fruit 2.

## Phase 3.

Dès que le fruit 2 est bloqué contre la plaque d'appui 3, le vérin 16 est actionné, sa tige mobile 16a se déplace du côté du fruit 2 et la gouge 14 qu'elle porte, pénètre de quelques centimètres dans le fruit et découpe une carotte de pulpe.

## Phase 4.

Au terme de sa pénétration, le vérin 16 est actionné en sens inverse en entraînant la gouge 14. Celle-ci sort du fruit en portant à l'intérieur la carotte de pulpe qui vient d'être prélevée et se déplace sur une distance sensiblement égale à la distance parcourue lors de sa pénétration dans le fruit. Pendant le mouvement de retrait de la gouge 14, le talon de la carotte qui comporte la peau du fruit vient en butée sur l'extrémité libre de la tige 25 coaxiale à la gouge, laquelle tige 25 est immobile pendant cette phase. Le mouvement relatif de la gouge 14 et de la tige 25 provoque en partie la sortie de la carotte de pulpe pour présenter cette dernière au-dessus du prisme 34a du réfractomètre électronique 34.

## Phase 5.

Dès que ladite partie de pulpe est dans cette position, le vérin 36 est actionné, sa tige mobile 36a se déplace verticalement du côté du prisme 34a et descend jusqu'à quelques dixièmes de millimètres du prisme en écrasant la partie de carotte émergeant de la gouge 14, en expulsant le jus contenu dans la pulpe, lequel jus se dépose sur le prisme du réfractomètre électronique 34.

## Phase 6.

Le vérin 16 est de nouveau actionné pour que sa tige mobile 16a se déplace du côté du fruit 2 en entraînant la gouge 14. Celle-ci, qui contient à l'intérieur le talon de la carotte traverse le piston 37 en empruntant l'orifice 37a et pénètre de nouveau dans le fruit toujours immobilisé, par le même trou fait lors du prélèvement de la carotte.

## Phase 7.

Lorsque la gouge 14 est parvenue en fin de course, le vérin 27 est actionné, sa tige mobile 27a portant la tige 25 se déplace à travers la gouge du côté du fruit 2 jusqu'à atteindre la face de la plaque d'appui 3 sur laquelle le fruit est en appui, donc jusqu'à la périphérie du fruit 2.

## Phase 8.

Le vérin 16 est actionné pour revenir en position "rentrée" en entraînant la gouge 14 hors du fruit. Le talon de la carotte, toujours à l'intérieur de la gouge, vient en butée sur la tige 25 en position "sortie" et reste ainsi dans le fruit en rebouchant le trou pratiqué lors du prélèvement de la carotte.

## Phase 9.

Le vérin 27 est actionné pour revenir à sa position "rentrée" en entraînant la tige 25. Simultanément, la rentrée du vérin 4 libère le fruit sur le convoyeur.

## Phase 10.

Le vérin 36 revient en position "rentrée", sa tige mobile 36a remonte en entraînant le piston 37.

**Phase 11.**

Au terme de la phase 10, la vanne 44 est actionnée en position ouverte et éjecte par le gicleur 45 un jet d'eau sur le prisme 34a du réfractomètre électronique pendant quelques dixièmes de secondes pour rincer ledit prisme.

**Phase 12.**

Au terme de la phase 11 la vanne 49 est actionnée en position ouverte et éjecte par le gicleur 50 un jet d'air sur le prisme 34a pour expulser par soufflage les déchets de pulpe et autres impuretés et sécher la surface du prisme 34 afin de le mettre en condition pour une nouvelle analyse.

**Phase 13.**

Pendant le déroulement des phases précédemment décrites 4 à 9, le réfractomètre électronique 34 analyse la teneur en sucre du jus extrait lors de la phase d'écrasement de la pulpe. Le résultat de cette analyse transformée en signal est rentré dans l'automate programmable 9 qui exécute ce signal en fonction du seuil préalablement programmé dans le réfractomètre 34, par exemple une valeur de 9 Brix. Si le taux de sucre découlant de l'analyse réfractométrique est supérieure à ce seuil, le fruit 2 présente les qualités gustatives requises et est dirigé dans le sens de la flèche F1, vers une station de calibrage et de conditionnement ou vers un stockage. Si le taux de sucre est inférieur audit seuil, l'automate 9 actionne le vérin 55 dont la tige mobile 55a se déplaçant transversalement au convoyeur 1 pousse le fruit 2 dans le sens de la flèche F2, du côté du plan incliné 56 pour le déposer dans un bac de réception destiné à recevoir les fruits qui ne présentent pas lesdites qualités gustatives.

Du fait que plusieurs seuils peuvent être programmés, le dispositif peut être équipé de plusieurs vérins d'éjection 55 pour stocker les fruits en fonction de leur teneur en sucre.

Le cycle de fonctionnement est terminé.

Le fonctionnement de l'installation se poursuit par cycles successifs, chaque fois qu'un fruit 2 entre dans le champ de la cellule 63, 64.

Bien entendu, sans sortir du cadre de l'invention, les parties qui viennent d'être décrites à titre d'exemple non limitatif, peuvent être remplacées par des parties équivalentes remplissant la même fonction.

**Revendications**

1. Dispositif pour sélectionner en continu des fruits (2) d'une même variété en fonction de leur teneur en sucre, comportant, en combinaison:
- des moyens pour déplacer les fruits et pour les présenter un à un devant un poste (3, 4, 14, 16) pour prélever dans chaque fruit une carotte de pulpe;
- des moyens (36, 37) pour extraire de la pulpe le jus qu'elle contient;
- des moyens (34, 34a) pour analyser le jus et pour déterminer sa teneur en sucre;
- des moyens (9) pour comparer le résultat de chaque analyse à au moins un seuil prédéterminé correspondant à la teneur en sucre minimale au dessous duquel les fruits sont rejetés;
- des moyens (55, 56) pour évacuer les fruits dont la teneur en sucre se situe au-dessous dudit seuil;
- et des moyens (1) pour diriger les fruits sélectionnés vers un stockage ou une station de calibrage de conditionnement.

2. Dispositif selon la revendication 1, caractérisé en ce qu'il comporte des moyens (14, 16, 25, 27) pour replacer la pulpe dans le fruit (2) après l'extraction du jus.

3. Dispositif selon la revendication 1, dont les fruits (2) sont déplacés par un convoyeur (1), caractérisé en ce qu'il comporte un détecteur de présence (63, 64) situé sur le trajet des fruits (2) et à proximité de moyens pour immobiliser temporairement les fruits, consistant en une plaque d'appui (3) fixée le long d'un des bords latéraux (1b) du convoyeur (1) et d'un organe de blocage (5) porté par la tige mobile d'un vérin (4) pour être déplacée devant et perpendiculairement à ladite plaque d'appui (3) et transversalement au convoyeur (1) pour bloquer un à un les fruits (2) contre la plaque d'appui (3).

4. Dispositif selon l'une quelconque des revendications 1 à 3, caractérisé en ce que la plaque d'appui (3) est percée (3b) pour permettre le passage desdits moyens pour prélever une carotte de pulpe, lesquels moyens se composent d'une gouge (14) constituée par un tube dont le bord périphérique de son extrémité libre (14a) est affûté en biseau pour pouvoir pénétrer dans le fruit (2) et prélever une partie de pulpe ainsi que la peau du fruit, laquelle gouge (14) est portée par la tige mobile d'un vérin à double effet (16) et se déplace parallèlement au-dessus du tapis (1a) du convoyeur (1).

5. Dispositif selon la revendication 4, caractérisé en ce qu'il comporte une tige-poussoir (25) montée coulissante dans ladite gouge (14), laquelle tige-poussoir (25) est portée par la tige mobile d'un vérin à double effet (27) pour se déplacer dans la gouge (14) coaxialement à celle-ci pour, d'une part, faire sortir de la gouge une partie de la carotte de pulpe pour extraire le jus qu'elle contient et, d'autre part, replacer, après l'extraction du jus, la carotte dans le trou pratiqué dans le fruit lors du prélèvement de la carotte.

6. Dispositif selon l'une quelconque des revendications 4 et 5, caractérisé en ce que, en position "rentrée" de la tige mobile et du vérin (16, 27), la position relative de la tige (25) et de la gouge (14) est telle que la pulpe d'une partie de la carotte est maintenue momentanément au-dessus du prisme (34a) d'un réfractomètre (34).

7. Dispositif selon la revendication 6, caractérisé en ce qu'il comporte au-dessus du prisme (34a) du réfractomètre (34) un pressoir constitué par un vérin à double effet (36), dont la tige mobile comporte à son extrémité libre un piston (37) destiné à presser la pulpe du fruit pour extraire le jus, lequel piston se déplace dans le sens vertical pour s'arrêter en position basse à proximité dudit prisme (34a).

8. Dispositif selon la revendicaton 7, caractérisé en ce que ledit piston (37) est percé (37a) dans le sens transversal pour permettre le passage de la gouge (14) pour replacer la carotte dans le fruit (2) après l'extraction du jus.

9. Dispositif selon l'une quelconque des revendications 1 à 8, caractérisé en ce qu'il comporte des moyens pour rincer le prisme (34a) du réfractomètre (34) consistant en une vanne télécommandée (44) comportant un gicleur (45) dirigé vers ledit prisme (34a) et dont l'extrémité se situe à proximité de celui-ci, laquelle vanne (44) est reliée à un circuit d'eau sous pression (46) pour délivrer et projeter sur le prisme (34a) un jet d'eau après chaque analyse.

10. Dispositif selon l'une quelconque des revendications 1 à 8, caractérisé en ce qu'il comporte des moyens pour nettoyer et sécher le prisme (34a) du réfractomètre (34) consistant en une vanne télécommandée (49) comportant un gicleur (50) dirigé vers ledit prisme (34a) et dont l'extrémité se situe à proximité de celui-ci, laquelle vanne (49) est reliée à un circuit d'air comprimé (51) pour délivrer et souffler sur le prisme (34a) un jet d'air après le rinçage.

11. Dispositif selon l'une quelconque des revendications 1 à 10, caractérisé en ce que les moyens pour écarter les fruits (2), dont la teneur en sucre n'atteint pas ledit seuil, consistent en au moins un vérin (55) dont la tige mobile comporte à son extrémité libre un poussoir (56), lequel poussoir se déplace transversalement et parallèlement au-dessus du convoyeur (1) pour pousser les fruits (2) sur un plan incliné (56) débouchant dans un bac de réception.

12. Dispositif selon l'une quelconque des revendications 1 à 11, dont les vérins comportent des capteurs de position et sont pilotés, caractérisé en ce que le détecteur de présence (63, 64), les capteurs de position (5, 6, 17, 18, 28, 29, 38, 39) et les pilotes des vérins (4, 16, 27, 36), le réfractomètre (34), les vannes d'eau (44) et d'air (49) pour rincer et pour nettoyer et sécher le prisme (34a) du réfractomètre (34) et le vérin d'éjection (55), sont reliés à une unité de commande (9) qui reçoit les signaux transmis par les différents appareils composant l'installation et en fonction d'un programme de fonctionnement préétabli, délivre des signaux de sortie pour commander lesdits appareils selon un cycle de fonctionnement.

**Patentansprüche**

1. Vorrichtung zum kontinuierlichen Aussortieren von Früchten (2) ein- und derselben Sorte nach ihrem Zuckergehalt, umfassend in Kombination:
- Mittel zum Fortbewegen der Früchte und zum stückweisen Anordnen vor einem Stand (3, 4, 14, 16) zur Entnahme einer Pulpenbohrprobe;
- Mittel (36, 37) zur Extraktion von in der Pulpe enthaltenem Saft;
- Mittel (34, 34a) zum Analysieren des Saftes und zum Bestimmen seines Zuckergehalts;
- Mittel (9) zum Vergleichen des Resultats jeder Analyse mit mindestens einem vorbestimmten Schwellenwert entsprechend dem minimalen Zuckergehalt, unterhalb welchem die Früchte verworfen werden;
- Mittel (55, 56) zum Entfernen der Früchte, deren Zuckergehalt unterhalb des Schwellenwerts liegt;
- und Mittel (1) zum Weiterleiten der aussortierten Früchte zu einem Lagerplatz oder einer Klassierungs-Verpackungs-Station.

2. Vorrichtung nach Anspruch 1, dadurch gekennzeichnet, daß sie Mittel (14, 16, 25, 27) zum Wiedereinsetzen der Pulpe in die Frucht (2) nach Extrakton des Saftes umfaßt.

3. Vorrichtung nach Anspruch 1, bei welcher die Früchte (2) von einem Förderer (1) weiterbewegt werden, dadurch gekennzeichnet, daß sie einen an der Bahn der Früchte (2) und in Nähe der Mittel zum vorübergehenden Stoppen der Früchte befindlichen Anwesenheitsdetektor (63, 64) umfaßt, welcher aus einer Stützplatte (3), die entlang einer der seitlichen Borde (1b) des Förderers (1) befestigt ist, und aus einem Blockierorgan (5), das von der beweglichen Stange eines Stelltriebs (4) getragen ist, um vor und senkrecht zu der Stützplatte (3) und quer zum Förderer (1) zwecks Blockierens der Früchte (2) einer nach der anderen an der Stützplatte (3) verschoben zu werden, besteht.

4. Vorrichtung nach einem der Ansprüche 1 bis 3, dadurch gekennzeichnet, daß die Stützplatte (3) durchbrochen (3b) ist, um den Durchtritt der Mittel zum Entnehmen einer Pulpenbohrprobe zu ermöglichen, welche Mittel durch ein Hohlmesser (14) gebildet sind, das aus einem Rohr besteht, dessen Umfangsrand seines freien Endes (14a) zum Eindringen in die Frucht (2) und Entnehmen eines Pulpenteils sowie der Schale der Frucht wechselseitig schräggeschliffen ist, welches Hohlmesser von der beweglichen Stange eines Stelltriebs (16) mit Doppelwirkung getragen ist und sich oberhalb des Bandes (1a) des Förderers (1) parallel verschiebt.

5. Vorrichtung nach Anspruch 4, dadurch gekennzeichnet, daß sie eine gleitend im Hohlmesser (14) gelagerte Stößelstange (25) umfaßt, welche Stößelstange (25) von der beweglichen Stange eines Stelltriebs (27) mit Doppelwirkung getragen ist, zwecks Hin- und Herbewegens im Hohlmesser (14) koaxial zu diesem, um einerseits einen Teil der Pulpenbohrprobe zum Extrahieren des darin enthaltenen Saftes aus dem Hohlmesser hinauszulassen und anderseits die Bohrprobe nach Extraktion des Saftes in das in der Frucht bei Entnahme der Bohrprobe entstandene Loch zurückzugeben.

6. Vorrichtung nach einem der Ansprüche 4 und 5, dadurch gekennzeichnet, daß in "eingezogener" Position der beweglichen Stange und des Stelltriebs (16, 27) die Relativposition zwischen Stange (25) und Hohlmesser (14) derart ist, daß die Pulpe eines Teils der Bohrprobe einen Augenblick über dem Prisma (34a) eines Refraktometers (34) gehalten wird.

7. Vorrichtung nach Anspruch 6, dadurch gekennzeichnet, daß sie oberhalb des Prismas (34a) des Refraktometers (34) eine Presse umfaßt, die durch einen Stelltrieb (36) mit Doppelwirkung gebildet ist, dessen bewegliche Stange an ihrem freien Ende einen Kolben (37) aufweist, der zum Pressen der Pulpe der Frucht zwecks Extraktion des Saftes bestimmt ist, wobei sich der Kolben in vertikaler Richtung bewegt, um in der unteren Position in der Nähe des Prismas (34a) zu halten.

8. Vorrichtung nach Anspruch 7, dadurch gekennzeichnet, daß der Kolben (37) in Querrichtung unterbrochen (37a) ist, um den Durchtritt des Hohlmessers (14) zwecks Zurückbringens der Bohrprobe in die Frucht (2) nach Extraktion des Saftes zu ermoglichen.

9. Vorrichtung nach einem der Ansprüche 1 bis 8, dadurch gekennzeichnet, daß sie Mittel zum Spülen des Prismas (34a) des Refraktometers (34) umfaßt, die aus einem ferngesteuerten Ventil (44) mit zum Prisma (34a) gerichteter Spritzdüse (45) bestehen, deren Ende sich in dessen Nähe befindet, welches Ventil (44) an einen Druckwasser-Kreislauf (46) angeschlossen ist, um nach jeder Analyse einen Wasserstrahl abzugeben und auf das Prisma (34a) zu schleudern.

10. Vorrichtung nach einem der Ansprüche 1 bis 8, dadurch gekennzeichnet, daß sie Mittel zum Reinigen und Trocknen des Prismas (34a) des Refraktometers (34) umfaßt, die aus einem ferngesteuerten Ventil (49) mit zum Prisma (34a) gerichteter Sprühdüse (50) bestehen, deren Ende sich in dessen Nähe befindet, welches Ventil (49) an einen Druckluft-Kreislauf (51) angeschlossen ist, um nach dem Spülen einen Luftstrahl abzugeben und auf das Prisma (34a) zu blasen.

11. Vorrichtung nach einem der Ansprüche 1 bis 10, dadurch gekennzeichnet, daß die Mittel zum Trennen der Früche (2), deren Zuckergehalt den Schwellenwert nicht erreicht, aus mindestens einem Stelltrieb (55) bestehen, dessen bewegliche Stange an ihrem freien Ende einen Stößel (56) umfaßt, welcher Stößel sich quer und parallel über dem Förderer (1) bewegt, um die Früchte (2) auf eine in einem Aufnahmebehälter mündende geneigte Ebene (56) zu stoßen.

12. Vorrichtung nach einem der Ansprüche 1 bis 11, bei welcher die Stelltriebe Positionsaufnehmer umfassen und gesteuert sind, dadurch gekennzeichnet, daß der Anwesenheitsdetektor (63, 64), die Positionsdetektoren (5, 6, 17, 18, 28, 29, 38, 39) und die Stelltriebsteuerungen (4, 16, 27, 36), das Refraktometer (34), die Wasser- (44) und Luft- (49)-Ventile zum Spülen und Reinigen und Trocknen des Prismas (34a) des Refraktometers (34) und der Ejektorstelltrieb (55) mit einer Steuereinheit (9) verbunden sind, die die von den verschiedenen, die Installation bildenden Apparaten gesendeten Signale empfängt und als Funktion eines zuvor erstellten Funktionsprogramms Ausgangssignale zum Steuern der Apparate entsprechend einem Funktionszyklus abgibt.

## Claims

1. Device for continuously selecting fruits (2) of a same variety, as a function of their sugar content, comprising in combination:
   - means for moving the fruits and for presenting them one by one before a station (3, 4, 14, 16) in order to remove from each fruit a sample of pulp;
   - means (36, 37) for extracting from the pulp the juice contained therein;
   - means (34, 34a) for analysing the juice and for determining its sugar content;
   - means (9) for comparing the result of each analysis to at least a predetermined threshold corresponding to the minimum sugar content below which the fruits are rejected;
   - means (55, 56) for removing the fruits, the sugar content of which is below said threshold;
   - and means (1) for directing the selected fruits towards storage means or towards package-grading station.

2. Device according to claim 1, characterized in that it comprises means (14, 16, 25, 27) for replacing the pulp into the fruit (2) after extraction of the juice.

3. Device according to claim 1, in which the fruits are moved by a conveyor (1), characterized in that it comprises a presence detector (63, 64) situated on the path of the fruits (2) and close to means for temporarily immobilizing the fruits, constituted of a supporting plate (3) secured along one of the side edges (1b) of the conveyor (1) and along a blocking member (5) carried by the morable rod of a jack (4) in order to be moved in front of and perpendicularly to said supporting plate (3) and transversally to said conveyor (2) in order to block the fruits (2) one by one against the supporting plate (3).

4. Device according to any one of claims 1 to 3, characterized in that the supporting plate (3) is perforated (3b) in order to allow the passage of said means for removing a sample of pulp, said means are composed of a gouge (14) constituted by a tube, of which the peripheral edge of its free end (14a) is bevelled in order to penetrate into the fruit (2) and to remove a piece of pulp as well as the skin of the fruit, said gouge (14) is carried by the movable rod of a doubleacting jack (16) and moves in parallel to and above the belt (1a) of the conveyor (1).

5. Device according to claim 4, characterized in that it comprises a push-rod (25) mounted for sliding inside said gouge (14), said push-rod (25) is carried by the movable rod of a double-acting jack (27) to move into the gouge (14) coaxially thereto, in order on the one hand, to bring out of the gouge, part of the pulp sample, and thus extract the juice contained therein, and on the other hand, to replace, after extraction of the juice, the sample into the hole made in the fruit during removal of the sample.

6. Device according to any one of claims 4 and 5, characterized in that, in a retracted position of the movable rod and of the jack (16,27), the relative position of the rod (25) and of the gouge (14) is such that the pulp of a part of the sample is held temporarily abore the prism (34a) of a refractometer (34).

7. Device according to claim 6, characterized in that it comprises, above the prism (34a) of the refractometer (34), a press member constituted by a doubleacting jack (36), of which the morable rod comprises at its free end a piston (37) designed to press the fruit pulp in order to extract the juice therefrom, said piston moving in the vertical direction and stopping in a low position near said prism (34a).

8. Device according to claim 7, characterized in that said piston (37) is perforated (37a) in the transversal direction to allow the passage of the gouge (14) for replacing the sample into the fruit (2) after extraction of the juice.

9. Device according to any one of claims 1 to 8, characterized in that it comprises means for rinsing the prism (34a) of the refractometer (34) constituted of a telecontrolled valve (44) comprising a spraying nozzle (45) directed towards said prism (34a), the end of which being situated near said prism, which valve (44) is connected to a pressurized water circuit (46) for delivering and spraying onto the prism (34a) a jet of water after each analysis.

10. Device according to any one of claims 1 to 8, characterized in that it comprises means for cleaning and drying the prism (34a) of the refractometer (34) constituted of a telecontrolled valve (49) comprising a spraying nozzle (50) directed towards said prism (34a), and the end of which being situated near said prism, said valve (49) is connected to a compressed air circuit (51) for delivering and blowing onto the prism (34a) an air jet after rinsing.

11. Device according to any one of claims 1 to 10, characterized in that the means for disposing of fruits (2), the sugar content of which is below said threshold, consist at least in a jack (55) of which the movable rod comprises at its free end a pusher (56), which pusher mores transversally and in parallel above the conveyor (1) to push the fruits (2) on an inclined plane (56) issuing into a receiving container,

12. Device according to any one of claims 1 to 11, of which the jacks comprise position sensors and are controlled, characterized in that the presence detectors (63, 64), the position sensors (5, 6, 17, 18, 28, 29, 38, 39), and the controls of the jack (4, 16, 27, 36), the refractometer (34), the water (44) and air (49) valves for rinsing, cleaning and drying the prism (34a) of the refractometer (34) and the ejection jack (55), are connected to a control unit (9) which receives the signals transmitted by the different apparatus composing the installation and as a function of a predetermined program of operation, delivers output signals for controlling said apparatus according to a working cycle.